# EUROPEAN PATENT APPLICATION

(11) **EP 1 970 090 A1**
(43) Date of publication of application: **17.09.2008**
(21) Application number: 08250676.7
(22) Date of filing: 28.02.2008
(51) Int. Cl.: A61M 25/01, A61M 25/00, A61N 1/05

(54) **A formable stylet**

(30) Priority: 12.03.2007 US 906722
(71) Applicant: CathRx Ltd, Eveleigh, NSW 1430 (AU)
(72) Inventor: Anderson, Neil Lawrence, Roseville New South Wales 2069 (AU); Partlett, Matthew, Allawah New South Wales 2218 (AU)
(74) Representative: Nettleton, John Victor

(57) **Abstract**

A formable stylet 10 includes an elongate body member 12 of a flexible material, the body member having a proximal end 14 and a distal end 16. The body member 12 has a distal portion 18 of reduced stiffness in comparison with the remainder of the body member 12, the distal portion 18 being able to be manipulated into a desired configuration.

## Description

### Field

This invention relates, generally, to a stylet and, more particularly, to a formable stylet. The stylet is intended particularly, but not necessarily exclusively, for use with a catheter.

### Background

In cardiovascular procedures using catheters, the catheter is, generally, inserted through the femoral vein of a patient and is steered to a site in a patient's heart. In the heart, the catheter is used for diagnostic or therapeutic purposes. Naturally, the catheter comes into contact with bodily fluids of the patient. Thus, the catheter is used once only and is then disposed of as sterilisation for re-use is too complicated.

The applicant has developed a modular catheter system whereby, if desired, certain components can be used a number of times. Generally, only those components which do not come into contact with the patient's bodily fluids can be re-used. Thus, an item such as an internal stylet of the catheter assembly can, in certain circumstances, be reused.

The modular arrangement of the applicant's catheter assembly means that there is a separate handle component, a separate electrode sheath component and a separate stylet, whether a deflectable stylet or a fixed curve stylet. The applicant has also devised a handle which does not have electrical conductors and connectors and is a low-cost component.

Because of the expensive nature of catheters, it would be desirable to have a low cost catheter assembly which, if it is to be disposed of, would not result in significant cost. Generally, with fixed curve stylets for catheters, these stylets are made of an expensive material and, to dispose of them, results in significant cost. Also, part of the cost of such stylets arises from the fact that it is difficult to machine or work the material of the stylet to form a fixed curve stylet.

### Summary

According to a first aspect of the invention, there is provided a formable stylet which includes
an elongate body member of a flexible material, the body member having a proximal end and a distal end; and
a distal portion of reduced stiffness in comparison with the remainder of the body member, the distal portion being able to be manipulated into a desired configuration.

By "flexible" is meant that the elongate body member is able to be bent into a desired shape but still has sufficient rigidity or stiffness to enable a catheter in which the stylet is received to be steered.

In use, a clinician is able to bend the distal portion manually into the desired shape for the clinician's purpose prior to insertion of the stylet into a lumen of an electrode sheath of a catheter.

The reduced stiffness of the distal portion of the body member may be provided by shaping the distal portion.

In an embodiment, the distal portion may be machined into a flattened shape. In another, preferred, embodiment, the distal portion may be worked into the flattened shape. More particularly, the distal portion may be worked into the flattened shape by rolling.

A remainder of the body member, proximally of the distal portion, may be of a cross-sectional shape to provide desired stiffness and torque characteristics. Thus, the remainder of the body member may be of substantially round cross-section of the desired diameter.

The body member may be of a low-cost, biocompatible material. The body member may be of a material selected from the group consisting of stainless steel, cobalt-chromium-nickel alloy (Elgiloy^{®}), nickel-cobalt base alloy (MP35N^{®}) and cobalt-nickel-chromium-molybdenum alloy (35N-LT^{®}). Other suitable flexible, biocompatible materials may also be used.

The distal portion of the body member may carry at least one radio opaque marker.

According to a second aspect of the invention, there is provided a catheter assembly which includes
a catheter handle;
an electrode sheath extending distally from the handle, the electrode sheath defining a lumen; and
a stylet, as described above, received in the lumen of the electrode sheath.

An attachment device may be mounted to the proximal end of the body member of the stylet, the attachment device including mounting formations which cooperate with complementary locating formations at the proximal end of the handle for securing the stylet with respect to the handle and the electrode sheath.

According to a third aspect of the invention, there is provided a method of fabricating a stylet, the method including
providing an elongate body member of a flexible material, the body member having a proximal end and a distal end; and
reducing the stiffness of a distal portion of the body member in comparison with the remainder of the body member to enable the distal portion to be manipulated into a desired configuration.

The method may include reducing the stiffness of the distal portion of the body member by shaping the distal portion of the body member.

In an embodiment, the method may include machining the distal portion of the body member. In another, preferred embodiment, the method may include working the distal portion of the body member. More particularly, the method may include working the distal portion of the body member by rolling.

The method may include selecting the material of the body member from the group consisting of stainless steel, cobalt-chromium-nickel alloy, nickel-cobalt base alloy and cobalt-nickel-chromium-molybdenum alloy.

The method may include applying at least one radio opaque marker to the distal portion of the body member. The at least one radio opaque marker may be applied by pad printing.

### Brief Description of Drawings

Fig. 1 shows a side view of a formable stylet, in accordance with an embodiment of the invention, in a first configuration;
Fig. 2 shows a side view of the stylet in a second configuration;
Fig. 3 shows a three dimensional view of the stylet in its first configuration;
Fig. 4 shows a three dimensional view of the stylet in its second configuration; and
Fig. 5 shows a three dimensional view of a catheter assembly in accordance with another embodiment of the invention.

### Detailed Description of Exemplary Embodiment

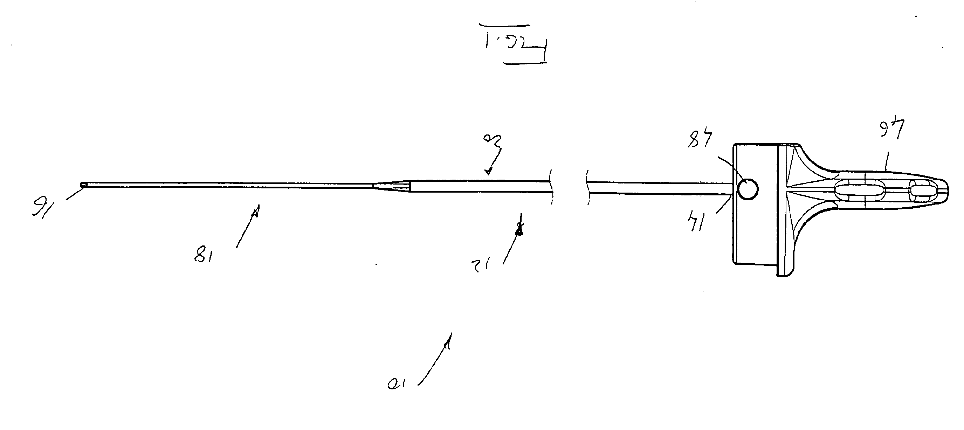

Referring initially to Figs. 1 to 4 of the drawings, a formable stylet in accordance with an embodiment of the invention is illustrated and is designated generally by the reference numeral 10. The stylet 10 comprises an elongate body member 12 having a proximal end 14 and a distal end 16. The body member 12 is of a flexible material.

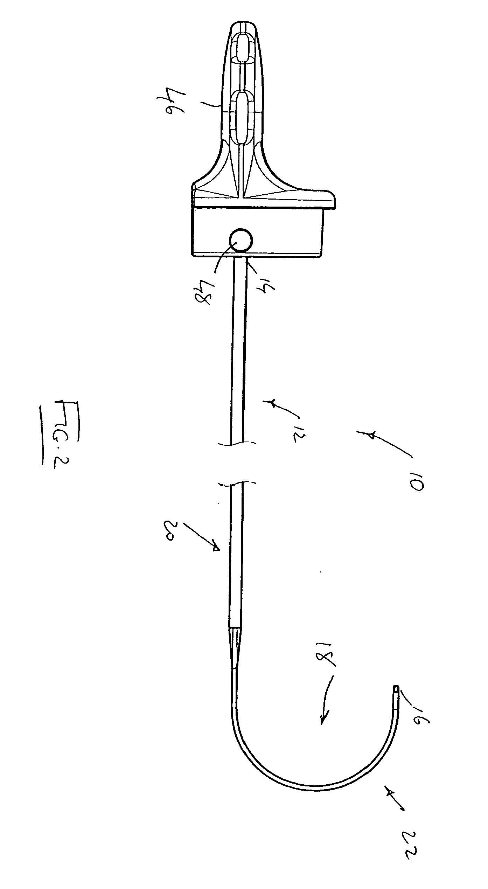

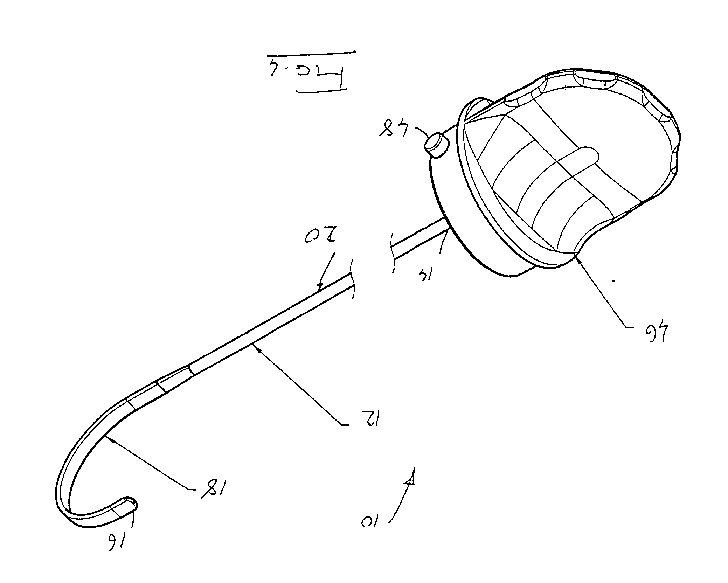

The stylet 10 has a shaped distal portion 18 to provide a region of reduced stiffness. The distal portion 18 is shaped, more particularly, flattened, to be of reduced bending stiffness in comparison with the remainder or proximal portion 20 of the body member 12 so that the distal portion 18 is able to be manipulated by a clinician into a desired configuration, such as a curved configuration as shown at 22 in Figs. 2 and 4 of the drawings.

While the distal portion 18 of the body member 12 may be flattened by machining, it is preferred and more cost effective to form the distal portion 18 by working the distal portion 18. More particularly, the distal portion 18 is formed into its flattened shape by a rolling operation.

The material from which the body member 12 is made is selected to have similar bending and torsional stiffness to a fixed curve Nitinol stylet. Further, the distal portion 18 of the body member 12 is flattened so that it has a similar bending stiffness to a machined distal portion of a fixed curved Nitinol stylet or to a conventional, commercial electro-physiological catheter. The distal portion 18 of the body member 12 is rolled flat so that it has a bending stiffness which is approximately 20%-25% of that of the remainder 20 of the body member 12.

Thus, for example, the material from which the body member 12 is made is selected to have a bending stiffness of the distal portion 18 of approximately 1 x 10⁻⁴ Nm². The remainder or proximal portion 20 of the body member 12 of the stylet 10 has a bending stiffness of approximately 4 x 10⁻⁴ Nm² - 5 x 10⁻⁴ Nm² and, more particularly, a bending stiffness of about 4.5 x 10⁻⁴ Nm². The proximal portion 20 of the body member of the stylet 10 has a torsional stiffness of approximately 3 x 10⁻⁴ Nm² - 4 x 10⁻⁴ Nm² and, more particularly, approximately 3.5 x 10⁻⁴ Nm².

It will be appreciated that the material selected for the body member 12 needs to be biocompatible. The materials from which the body member 12 could be made include a surgical grade stainless steel, such as 304 stainless steel, a cobalt-chromium-nickel alloy such as that sold under the registered trade mark "Elgiloy", a nickel-cobalt base alloy such as that sold under the registered trade mark "MP35N" or a cobalt-nickel-chromium-molybdenum alloy such as that sold under the registered trade mark "35N-LT'. Other suitable biocompatible materials could also be used.

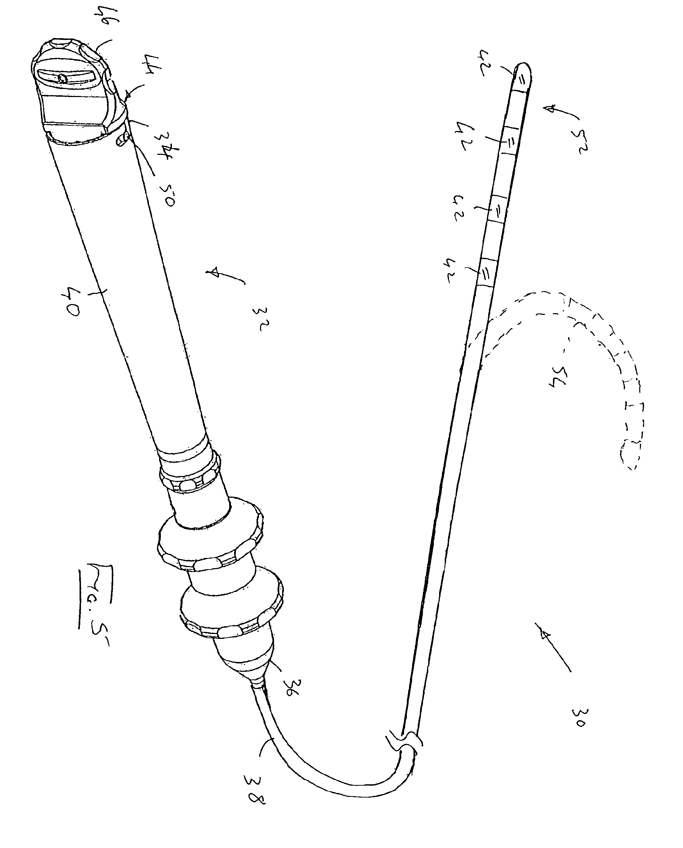

The formable stylet 10 is intended for use with a catheter assembly 30 (Fig. 5). The catheter assembly 30 is, generally, used in cardiovascular applications and comprises a catheter handle 32 having a proximal end 34 and a distal end 36. An electrode sheath 38 extends from the distal end 36 of the catheter handle 32. The electrode sheath 38 has an inner tubular member defining an unimpeded lumen in which the formable stylet 10 is received.

The handle 32 of the catheter assembly 30 comprises a hollow body 40 through which electrical conductors for electrodes 42 of the electrode sheath 38 pass, passing out of an opening 44 at the proximal 34 of the handle body 32. Thus, the handle body 32 is a low cost, disposable item.

To secure the formable stylet 10 in position relative to the handle 32 and the electrode sheath 38, an attachment device in the form of a mounting knob 46 is attached to the proximal end 14 of the elongate body 12. The mounting knob 46 is of a suitable low cost synthetic plastics material, such as polycarbonate, and is secured to the proximal end 14 of the elongate body 12 by adhesive such as an epoxy. The mounting knob 46 includes a pair of opposed, outwardly extending mounting formations, in the form of pins, 48, only one of which is shown in Fig. 5 of the drawings. The pins 48 engage complementary receiving formations, in the form of a pair of opposed L-shaped slots 50, at the proximal end 34 of the handle 32, bayonet fashion. This locks the formable stylet 10 in position relative to the catheter assembly 30.

The electrode sheath 38 has a plurality of spaced electrodes 42 at the distal portion 52 of the electrode sheath 38. It is intended that the electrode sheath 38 will also be a low cost, disposable item. Thus, the electrodes 42 may not have radio opaque markers associated with them. To enable a clinician to determine the position of the electrodes 42 in the patient's body once the electrode sheath 38 has been inserted into the patient's body, the flattened portion 18 of the body member 12 of the formable stylet 10 carries radio opaque markers 56. The radio opaque markers 56 are applied, for example, by pad printing. These radio opaque markers 56, once the stylet 10 has been inserted into the lumen of the electrode sheath 38, lie beneath the electrodes 42 in register with the electrodes 42. This enables a clinician to determine the position of the electrodes 42 under a fluoroscope.

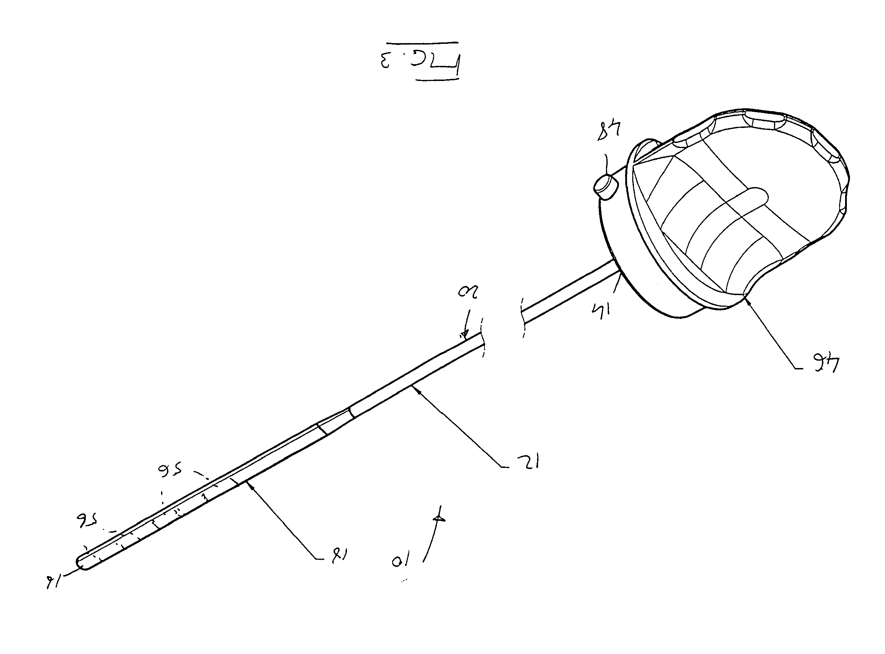

In use, the formable stylet 10 is provided with the distal portion 18 in an unformed, straight shape. This is shown more clearly in Figs. 1 and 3 of the drawings. When a clinician desires to form the distal portion 18 into a desired shape, the clinician bends the distal portion 18 into that shape. For example, the clinician bends the distal portion into a re-entrantly curved shape as shown in Figs. 2 and 4 of the drawings. The material from which the body member 12 is made, while being flexible, has limited resilience so that, once the shape has been formed in the distal portion 18, the material of the body member 12 causes that shape to be retained. This is so even after the body member 12 has been inserted into the lumen of the electrode sheath 38. Thus, a distal part 52 of the electrode sheath 38 is bent into the corresponding shape as shown in dotted lines at 54 in Fig. 5 of the drawings.

Because the formable stylet 10 is made of one of the materials selected above, it is a relatively low cost item. Thus, it can be disposed of after a single use without significant expense. It is therefore an advantage of the invention that a formable stylet 10 is provided which is a low cost, one-use device. This, in addition, means that the catheter assembly 30 is, similarly of a one-use, disposable nature. The provision of pad printed radio opaque markers 56 on the distal portion 18 of the formable stylet 12 further serves to provide a low cost solution to enable a clinician to determine the position of the electrodes within the patient's body. Further, the stylet 10 is versatile in that the clinician is able to shape the distal portion 18 into a desired shape for a particular application. The applicant has found that, surprisingly, the distal portion 18 is able to retain the shape into which it has been formed by the clinician even after the stylet 10 has been steered through the patient's vasculature.

It will be appreciated by persons skilled in the art that numerous variations and/or modifications may be made to the invention as shown in the specific embodiments without departing from the spirit or scope of the invention as broadly described. The present embodiments are, therefore, to be considered in all respects as illustrative and not restrictive.

## Claims

1. A formable stylet which includes
an elongate body member of a flexible material, the body member having a proximal end and a distal end; and
a distal portion of reduced stiffness in comparison with the remainder of the body member, the distal portion being able to be manipulated into a desired configuration.

2. The stylet of claim 1 the reduced stiffness of the distal portion of the body member is provided by shaping the distal portion.

3. The stylet of claim 2 in which the distal portion is formed into a flattened shape by one of machining and working.

4. The stylet of claim 3 in which the distal portion is worked into the flattened shape by rolling.

5. The stylet of any one of the preceding claims in which a remainder of the body member, proximally of the distal portion, is of a cross-sectional shape to provide desired stiffness and torque characteristics.

6. The stylet of any one of the preceding claims in which the body member is of a low-cost, biocompatible material selected from the group consisting of stainless steel, cobalt-chromium-nickel alloy, nickel-cobalt base alloy and cobalt-nickel-chromium-molybdenum alloy.

7. The stylet of any one of the preceding claims in which the distal portion of the body member carries at least one radio opaque marker.

8. A catheter assembly which includes
a catheter handle;
an electrode sheath extending distally from the handle, the electrode sheath defining a lumen; and
a stylet, as claimed in any one of the preceding claims, received in the lumen of the electrode sheath.

9. The assembly of claim 8 in which an attachment device is mounted to the proximal end of the body member of the stylet, the attachment device including mounting formations which cooperate with complementary locating formations at a proximal end of the handle for securing the stylet with respect to the handle and the electrode sheath.

10. A method of fabricating a stylet, the method including
providing an elongate body member of a flexible material, the body member having a proximal end and a distal end; and
reducing the stiffness of a distal portion of the body member in comparison with the remainder of the body member to enable the distal portion to be manipulated into a desired configuration.

11. The method of claim 12 which includes reducing the stiffness of the distal portion of the body member by shaping the distal portion of the body member.

12. The method of claim 11 which includes shaping the distal portion of the body member by one of machining and working.

13. The method of claim 15 which includes working the distal portion of the body member by rolling.

14. The method of any one of claims 10 to 13 which includes selecting the material of the body member from the group consisting of stainless steel, cobalt-chromium-nickel alloy, nickel-cobalt base alloy, and cobalt-nickel-chromium-molybdenum alloy.

15. The method of any one of claims 10 to 14 which includes applying at least one radio opaque marker to the distal portion of the body member.
